# EUROPEAN PATENT APPLICATION

(11) **EP 4 752 208 A1**
(43) Date of publication of application: **03.06.2026**
(21) Application number: 24216605.6
(22) Date of filing: 29.11.2024
(51) Int. Cl.: C12M 1/00, C12M 1/12, C12M 3/06

(54) **MICROFLUIDIC DEVICE FOR CULTURING CELLS**

(71) Applicant: Imec VZW, 3001 Leuven (BE)
(72) Inventor: CONDOR, Maria del Mar, 3000 Leuven (BE); BRAEKEN, Dries, 3401 Waasmont (BE)
(74) Representative: Winger

(57) **Abstract**

A method for forming a microfluidic device for culturing cells, the method comprising: providing a microfluidic device comprising a first chamber and a second chamber fluidically connected to the first chamber by a first porous structure comprising through holes for allowing fluid to pass through, said first porous structure physically separating the first chamber and the second chamber, wherein each of said chambers has photosensitive inner walls having a first property; and then irradiating the microfluidic device with light to which the photosensitive inner walls are sensitive, in such a way that the first porous structure acts as a mask during irradiation, thereby changing the surface property of the exposed surfaces and imparting a different surface property in the first chamber compared to the second chamber.

## Description

### Field of the Invention

The present invention relates to microfluidic devices, and more specifically to microfluidic devices for culturing cells.

### Background of the Invention

Microfluidic devices have revolutionized cell culture and tissue engineering by enabling precise control over cellular microenvironments at microscale dimensions. These devices typically comprise multiple chambers or channels that need to maintain distinct properties while allowing controlled interactions between them. However, creating and maintaining different properties in adjacent chambers remains a significant challenge, particularly when these chambers are fluidically connected.

A major limitation in current microfluidic technology is the difficulty of selectively modifying properties within specific regions of an assembled device. This challenge becomes particularly acute when different cell types or biological materials require different conditions for optimal function.

One example demonstrating these challenges is the development of blood-brain barrier (BBB) models using microfluidic platforms.

These devices often incorporate a porous membrane or mesh structure upon which endothelial cells form a monolayer, mimicking the selective permeability of the BBB. Beneath this layer, brain cells such as astrocytes and pericytes are embedded within a hydrogel matrix, creating a more physiologically relevant environment.

In these models, the maintenance of brain cell viability over extended periods is advantageous. This requires the hydrogel containing the cells to be adequately perfused with nutrients, oxygen, and other essential factors. Typically, microfluidic devices designed for BBB applications include multiple parallel channels: a central channel for the hydrogel and brain cells, and adjacent side channels that allow for the flow of culture medium. The medium flows through the side channels and diffuses into the hydrogel via openings or porous structures, supporting cell health and function.

A common challenge in the utilization of such microfluidic devices lies in the difficulty of assigning different biological functions to each chamber. For instance, enabling the incorporation of living cells in either 2D or 3D microenvironments across the various chambers is not straightforward. Meeting this challenge would permit the use of materials with differing chemical properties, such as wettability (hydrophilicity or hydrophobicity) in different microenvironments.

Despite advancements in microfluidic technology and device fabrication, creating distinct properties in different chambers of assembled microfluidic devices remains challenging. Current approaches often lack spatial selectivity, making it difficult to establish different microenvironments in connected chambers. Addressing this challenge would permit the development of reliable and versatile microfluidic platforms that can better replicate complex biological interfaces and enable advanced research in cell biology, tissue engineering, and drug development.

### Summary of the Invention

It is an object of embodiments of the present invention to provide a microfluidic device having different properties in different chambers and method for creating the same. This objective is accomplished by the aspects of the present invention.

In a first aspect, the present invention relates to a method for forming a microfluidic device for culturing cells, the method comprising:
(a) providing a microfluidic device comprising:
   o a first chamber; and
   o a second chamber fluidically connected to the first chamber by a first porous structure comprising through holes for allowing fluid to pass through, said first porous structure physically separating the first chamber and the second chamber,
   wherein each of said chambers has photosensitive inner walls having a first surface property; and then
(b) irradiating the microfluidic device with light to which the photosensitive inner walls are sensitive, in such a way that the first porous structure acts as a mask during irradiation, thereby changing the surface property of the exposed surfaces and imparting a different surface property in the first chamber compared to the second chamber.

In embodiments, the surface property may be selected from the chemical reactivity, the surface morphology (e.g., roughness), or the wettability. Such alterations allow to form surfaces that can accommodate different cell types or different materials at different locations of the devices.

Typically, the irradiation is performed in the direction from the first chamber to the second chamber so that all walls of the first chamber are entirely exposed and at most part of the walls of the second chamber are exposed. Typically, the first chamber has a (top) wall opposite to the first porous structure and this wall is transparent to said light.

The first porous structure blocks at least partially the said light from irradiating the second chamber. In embodiments, the first porous structure may let pass through its pores from 10 to 90%, preferably from 20 to 80% of said light hitting it.

In embodiments, step (b) may result in exposing to said light a larger proportion of the photosensitive inner walls of the first chamber than of the photosensitive inner walls of the second chamber. This allows imparting different surface property in different chambers.

In embodiments, each of the photosensitive inner walls may comprise a photosensitive coating, such as a photosensitive coating comprising photosensitive silane molecules. Using a photosensitive coating enables convenient light-based patterning of surface properties.

In embodiments, step (b) may degrade the photosensitive coating. Degradation of the coating by light exposure changes the surface property.

In embodiments, the photosensitive coating may be a hydrophobic coating such as a hydrophobic silane coating.

In embodiments, the photosensitive coating may be a hydrophilic coating or a reactive coating such as a amino-functionalized or a carboxy-functionalized silane coating.

In embodiments, step (b) may degrade a greater proportion of the photosensitive coating in the first chamber than in the second chamber. This results in a surface property difference between the chambers.

In embodiments, at least some of the photosensitive inner walls may comprise a semiconductor material, e.g., Si, on which the photosensitive coating is coated. The semiconductor provides a suitable substrate for the photosensitive coating. In particular, Si is easily functionalized with a photosensitive coating.

It is an advantage of embodiments of the present invention that utilizing a silicon-based microfluidic device offers more precise fabrication of small structures and greater control over surface properties compared to polymer-based devices.

In embodiments, the photosensitive inner walls of a first surface property may be of a first wettability (e.g., they may be hydrophobic) and wherein at least some, and preferably all, of the irradiated inner walls change their wettability (e.g., become hydrophilic) after step (b), and vice versa. This hydrophobic-hydrophilic patterning is useful for controlling fluid behavior, material placement, or hydrogel placement.

In embodiments, the light may have a wavelength in the range of from 100 to 400 nm, such as from 150 to 340 nm, or from 220 to 290 nm or from 240 to 270 nm. These wavelength range is typically effective for modifying the photosensitive coating.

In embodiments, the intensity and the time of the irradiation is such that a modification of the surface property is achieved with the used light source. For instance, the intensity may be from 5 to 50 mW/cm² or from 15 to 30 mW/cm². For instance, the irradiation time may be from 5 s to 90 minutes.

In embodiments, the microfluidic device may further comprise a third chamber separated from the second chamber by a second porous structure for retaining hydrogel and ensuring a fluidic connection between the second chamber and the third chamber. The third chamber allows for the flow of culture medium and for the diffusion of the culture medium in the second chamber. The first porous structure extends in the third chamber, thereby separating the third chamber into an upper part and a lower part that are fluidically connected.

In embodiments, the microfluidic device may further comprise a fourth chamber separated from the second chamber by a third porous structure for retaining hydrogel and ensuring a fluidic connection between the second chamber and the fourth chamber. The fourth chamber allows for the flow of culture medium and for the diffusion of the culture medium in the second chamber. The first porous structure extends in the fourth chamber, thereby separating the fourth chamber into an upper part and a lower part that are fluidically connected.

In embodiments, the second and third porous structures may be structurally different from the first porous structure.

In embodiments, the second porous structure and/or the third porous structure may comprise a plurality of pillars, wherein the spaces between the pillars ensure the fluidic connection between the second chamber and the third chamber and/or between the second chamber and the fourth chamber. The pillars help retain the content of the second chamber (e.g. a hydrogel) while allowing fluid exchange.

In embodiments, the method may further comprise:
(c) irradiating, after step (b), the third and/or fourth chamber without irradiating the second chamber, thereby changing a surface property (e.g., the wettability) of the irradiated surfaces and imparting a different surface property (e.g., wettability) in the third and/or fourth chamber compared to the second chamber. For instance, the third and fourth chamber may be hydrophobic before step (c) and become hydrophilic after step (c).

In embodiments, the wall of the second chamber opposite to the first porous structure (typically the bottom wall of the second chamber) may also extend laterally to form a wall of the third and fourth chambers (typically their bottom walls) and may be transparent to said light, and step (c) may be performed by masking the part of this wall overlapping with the second chamber without masking the part of this wall overlapping with the third and fourth chambers, then irradiating the device in the direction from the second chamber to the first chamber (typically from the bottom of the device).This can be achieved by using a photomask to mask said part. Preferably, the masking of said part is such that it blocks at least 60%, preferably at least 80%, even more preferably at least 90% and most preferably 100% of the light hitting said wall.

In embodiments, the method may further comprise:
(d) introducing, after step (b), a hydrogel into the second chamber. The hydrogel provides a 3D matrix for culturing cells.

In embodiments, step (d) may comprise:
- introducing a hydrogel precursor solution into the second chamber; and
- initiating polymerization of the hydrogel precursor solution to form the hydrogel.

In embodiments, initiating polymerization may comprise exposing the hydrogel precursor solution to a polymerization trigger. This encompasses any initiating factor that can start the polymerization process of the hydrogel precursor solution to form the hydrogel within the microfluidic device. In embodiments, the polymerization trigger may be selected from the group consisting of light, heat, and a chemical agent. Different triggers provide flexibility in hydrogel formation. Specific embodiments include ultraviolet light initiating polymerization in a photopolymerizable hydrogel, heat triggering gelation in a thermosensitive polymer like gelatin, or adding calcium ions to cross-link alginate chains chemically.

In embodiments, the first chamber may contain endothelial cells, and the second chamber may contain brain cells. This configuration is useful for blood-brain barrier models.

Any feature of the first aspect may be as correspondingly described in any of the other aspects.

In a second aspect, the present invention relates to a microfluidic device for culturing cells, the device comprising:
- a first chamber;
- a second chamber fluidically connected with the first chamber by a first porous structure comprising through holes for allowing fluid to pass through, said first porous structure physically separating the first chamber and the second chamber;
- each of said chambers having inner walls;
wherein a surface property in the first chamber is different compared to the second chamber.

In embodiments, the surface property may be selected from the chemical reactivity, the surface morphology (e.g., roughness), or the wettability. Such alterations allow to form surfaces that can accommodate different cell types or different materials at different locations of the devices.

In embodiments, the difference in surface property, e.g., in wettability, between the chambers is imparted by inner walls in one of the chambers comprising a photosensitive coating, such as, for instance, a photosensitive coating comprising photosensitive silane molecules.

In embodiments, the different surface property, e.g., wettability, of the first and second chambers may be obtainable by providing the first and second chambers with photosensitive inner walls, and by irradiating the microfluidic device with light to which the photosensitive inner walls are sensitive, in such a way that the first porous structure acts as a mask during irradiation. This light-based patterning process creates the surface property difference, e.g., wettability difference. In such embodiments, the photosensitive inner walls may comprise a photosensitive coating, such as, for instance, a photosensitive coating comprising photosensitive silane molecules. The photosensitive coating enables the light-based patterning.

In embodiments, the microfluidic device may further comprise a third chamber separated from the second chamber by a second porous structure for retaining hydrogel and ensuring a fluidic connection between the second chamber and the third chamber. The third chamber allows for the flow of culture medium and for the diffusion of the culture medium in the second chamber. The first porous structure extends in the third chamber, thereby separating the third chamber into an upper part and a lower part that are fluidically connected.

In embodiments, the microfluidic device may further comprise a fourth chamber separated from the second chamber by a third porous structure for retaining hydrogel and ensuring a fluidic connection between the second chamber and the fourth chamber. The fourth chamber allows for the flow of culture medium and for the diffusion of the culture medium in the second chamber. The first porous structure extends in the fourth chamber, thereby separating the third chamber into an upper part and a lower part that are fluidically connected.

In embodiments, the second porous structure and/or the third porous structure may comprise a plurality of pillars, wherein the spaces between the pillars ensure the fluidic connection between the second chamber and the third chamber and/or between the second chamber and the fourth chamber. The pillars help compartmentalize the hydrogel while allowing fluidic communication.

In embodiments, there may be a different surface property (e.g., wettability) in the third and/or fourth chamber compared to the second chamber. This allows for further customization of the microenvironment in each chamber.

In embodiments, the difference in surface property (e.g., wettability) in the third and/or fourth chamber compared to the second chamber is imparted by inner walls in one of the chambers comprising a photosensitive coating, such as, for instance, a photosensitive coating comprising photosensitive silane molecules.

In embodiments, the different surface property (e.g., wettability) of the third and/or fourth chambers may be obtainable by providing the third and fourth chambers with photosensitive inner walls, and by irradiating, after step (b), the third and/or fourth chamber without irradiating the second chamber, thereby changing the surface property (e.g., wettability) of the irradiated surfaces and imparting a different surface property (e.g., wettability) in the third and/or fourth chamber compared to the second chamber. This additional irradiation step patterns the surface property (e.g., wettability) in the third and fourth chambers independently. In such embodiments, the photosensitive inner walls of the third and fourth chambers may comprise a photosensitive coating, such as a photosensitive coating comprising photosensitive silane molecules. The photosensitive coating enables the light-based surface property (e.g., wettability) patterning.

In embodiments, each non-irradiated photosensitive inner wall of the first, second, third, and fourth chambers of the device may be hydrophobic and each irradiated photosensitive inner wall may be hydrophilic. This hydrophobic-hydrophilic patterning controls fluid behavior and allows, for instance, the introduction and maintaining of a hydrogel in the second chamber.

In embodiments, the first chamber may contain endothelial cells and the second chamber may contain brain cells. This setup mimics the blood-brain barrier.

Any feature of the second aspect may be as correspondingly described in any of the other aspects.

In a third aspect, the present invention relates to a system for culturing cells, the system comprising:
- the microfluidic device of any embodiment of the second aspect; and
- an injection tool configured to introduce a hydrogel into the second chamber.

The injection tool enables precise hydrogel placement.

In embodiments, the system may further comprise a control tool for managing fluid flow and environmental conditions within the microfluidic device. The tool may comprise one or more elements. Specific elements include syringe pumps for fluid delivery, valves for flow regulation, and temperature controllers for maintaining optimal cell culture conditions.

In embodiments, the system may further comprise a light source configured to irradiate the microfluidic device with light to which the photosensitive inner walls are sensitive, in such a way that the first porous structure acts as a mask during irradiation. The light source enables the selective wettability patterning between chambers.

Any feature of the third aspect may be as correspondingly described in any of the other aspects.

In a fourth aspect, the present invention relates to an intermediate in the formation of a microfluidic device for culturing cells, the device comprising:
- a first chamber;
- a second chamber fluidically connected with the first chamber by a first porous structure comprising through holes for allowing fluid to pass through, said first porous structure physically separating the first chamber and the second chamber;
- each of said chambers having photosensitive inner walls having a same surface property (e.g., wettability). Such intermediates can be shipped to the customer who can produce a difference between the wettability of the first and second chamber by irradiating the microfluidic device with light to which the photosensitive inner walls are sensitive, in such a way that the porous structure acts as a mask during irradiation.

Any feature of the fourth aspect may be as correspondingly described in any of the other aspects.

It is an advantage of embodiments of the present invention that selective surface chemistry modifications can impart some chambers with different surface properties than other chambers. For instance, they allow making some chambers more hydrophobic and others more hydrophilic, thereby allowing forming and keeping a hydrogel exclusively into the desired area. It is an advantage of embodiments of the present invention that the device's porous structure facilitates controlled communication between upper and lower chambers while maintaining distinct microenvironments, which is advantageous for accurately mimicking the blood-brain barrier. It is a further advantage of embodiments of the present invention that the versatile design can accommodate different cell types and tissues, allowing for multiple cell populations in separate compartments and the study of complex biological interfaces. It is an advantage of embodiments of the present invention that the method enables localized patterning of different surface chemistries, providing a flexible platform adaptable to a wide range of biomedical applications. It is a further advantage of embodiments of the present invention that the device can be integrated into a complete system with injection tools, control tools, and environmental management, enhancing its utility for various research and development purposes.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a schematic view of a method for forming a microfluidic device according to embodiments of the present invention.
Fig. 2 is a perspective view of a portion of a microfluidic device according to embodiments of the present invention.
Fig. 3 is a schematic view of a system for culturing cells according to embodiments of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top and over and the like in the description or the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", also used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be interpreted as being limited to devices consisting only of components A and B. It means that with respect to the present invention, the only relevant components of the device are A and B. The term "comprising" therefore covers the situation where only the stated features are present and the situation where these features and one or more other features are present. The word "comprising" according to the invention therefore also includes as one embodiment that no further components are present. When the word "comprising" is used to describe an embodiment in this application, it is to be understood that an alternative version of the same embodiment, wherein the term "comprising" is replaced by "consisting of", is also encompassed within the scope of the present invention.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some, but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The following terms are provided solely to aid in the understanding of the invention.

As used herein, and unless otherwise specified, the term "physically separating" is meant to mean " structurally segregating". It typically refers to a division into two or more chambers or regions within the microfluidic device. This is typically performed by a barrier structure, such as a porous structure, which prevents direct mixing or merging of the bulk volumes of the chambers while still allowing controlled fluid communication through defined pathways such as through-holes of a first porous substrates or spaces between pillars of a second or third porous substrate. The physical separation maintains distinct spatial compartments within the device while permitting selective transport of fluids, molecules, or other materials via the engineered passages in the separating structure. For example, the first chamber and second chamber are physically separated by the first porous structure, which prevents complete mixing of their contents while allowing controlled fluid exchange through, e.g., through holes, enabling the establishment of distinct microenvironments on either side of the barrier.

As used herein, and unless otherwise specified, the term "photosensitive inner walls" refers to the inner surfaces of the chambers within the microfluidic device that are sensitive to specific wavelengths of light. This can be achieved, for instance, by a coating or a treatment of the inner surface with a photosensitive material. Upon exposure to such light, these photosensitive materials undergo a chemical or physical change that alters the surface properties of the walls, such as their wettability. Examples of specific embodiments include inner walls coated with photosensitive silane molecules that degrade when irradiated with ultraviolet light in the wavelength range of from 100 to 400 nm. These photosensitive silanes can be categorized into different types based on their modification mechanism. A first type includes conventional silanes that undergo photo-oxidative degradation, such as silanes bearing a functional group such as an amino group or a carboxy group (e.g., (3-aminopropyl)trimethoxysilane) or silanes that bear a hydrophobic functional group such as a fluoroalkyl group (e.g., (tridecafluoro-1,1,2,2-tetrahydrooctyl)trimethoxysilane). The second type includes specifically designed photosensitive silanes incorporating photolabile protecting groups, such as silanes containing 2-nitrobenzyl ester groups that cleave under UV exposure to reveal carboxy groups, or aryldialkylchlorosilanes that decompose under visible light irradiation. As an example, (tridecafluoro-1,1,2,2-tetrahydrooctyl)trimethoxysilane creates a hydrophobic surface with initial water contact angles exceeding 90°. Upon exposure to UV light at, for instance, 254 nm with an intensity of 15-30 mW/cm² for 30-60 minutes in the presence of air, the fluoroalkyl chains undergo photo-oxidation and the contact angle drops below 90°. Similarly, aminosilanes such as (3-aminopropyl)trimethoxysilane may be used to make the initial surfaces reactive. Under UV exposure conditions (e.g., 254 nm, 15-30 mW/cm², 30-60 minutes), (3-aminopropyl)trimethoxysilane undergoes photo-oxidative degradation of the aminopropyl groups and reactivity drops. The photosensitive nature of these surfaces enables precise spatial control of surface properties through masked irradiation, with the non-irradiated regions maintaining their original surface characteristics while the exposed regions undergo controlled modification.

As used herein, and unless otherwise specified, the term "surface property" refers to any physical or chemical characteristic of a surface that can be modified by exposure to light, including but not limited to chemical reactivity, surface morphology (e.g., roughness), or wettability. In the context of the microfluidic device, surface properties determine how the inner walls of the chambers interact with fluids, cells, and other materials. These properties can be modified through exposure to specific wavelengths of light when the surfaces incorporate photosensitive materials. For example, exposure to ultraviolet light may alter the chemical groups present on a surface, changing its wettability from hydrophobic to hydrophilic. Surface properties may be characterized through various analytical techniques such as contact angle measurements for wettability, atomic force microscopy for surface roughness, or X-ray photoelectron spectroscopy for chemical composition. The phrase "changing the surface property of the exposed surfaces" refers therefore to any light-induced modification of these characteristics on surfaces that are not masked during irradiation. Similarly, the expression "imparting a different surface property in the first chamber compared to the second chamber" means that, following irradiation, at least one surface property of the inner walls in the first chamber, averaged over all its surfaces, is made different from the corresponding surface property of the inner walls in the second chamber, averaged over all its surfaces.

As used herein, and unless otherwise specified, the term "wettability" refers to the property of a solid surface that determines the degree to which it can be wet by a liquid. This is typically quantified by the contact angle between the liquid and the surface: a low contact angle indicates high wettability (hydrophilic surface), while a high contact angle indicates low wettability (hydrophobic surface). In the context of the microfluidic device, altering the wettability of the inner walls influences fluid flow, hydrogel retention, and cell attachment within the chambers. Specific embodiments include surfaces that are hydrophobic before irradiation and become hydrophilic after exposure to ultraviolet light, or vice versa. The phrase "changing the wettability of the exposed surfaces" refers therefore to the alteration of the surface wettability of the inner walls that are not masked during irradiation. Similarly, the expression "imparting a different wettability in the first chamber compared to the second chamber" means that, following irradiation, the wettability of the inner walls in the first chamber, averaged over all its surfaces, is made different from the wettability of the inner walls in the second chamber, averaged over all its surfaces. This differential wettability is achieved by selective irradiation. Irradiation changes the physical and/or chemical properties of the surface. This changes the wettability of the surface, leading to varied surface properties that can influence cell culture conditions and hydrogel retention. An example embodiment is where the first chamber becomes hydrophilic while the second chamber remains hydrophobic, facilitating distinct hydrogel retention.

As used herein, and unless otherwise specified, the expression "first porous structure acting as a mask during irradiation" refers to the function of the porous structure physically blocking or attenuating the passage of irradiation light to certain areas of the microfluidic device during the irradiation step. This selective masking results in differential exposure of the photosensitive inner walls, allowing only the surfaces not shielded by the porous structure to undergo a change in surface property (e.g., wettability).

In embodiments, the porous structure comprises a solid substrate with a plurality of through-holes distributed in a repetitive pattern, allowing fluid communication through the substrate. The solid substrate may include a top surface and a bottom surface, with the through-holes (e.g., straight through-holes) extending between these surfaces. In an example embodiment, the porous structure includes a solid substrate with through-holes that align with specific regions of the second chamber, permitting localized irradiation thereof. This arrangement can induce patterned surface property, such as patterned wettability, in the second chamber. The substrate may be made of any material and is preferably made of a CMOS-compatible material such as silicon, germanium, silicon nitride (SiN), or silicon oxide. These materials not only ensure compatibility with established fabrication processes but also provide desirable physical and chemical properties for integration into complex systems.

In some embodiments, the substrate may have a maximal thickness of less than 500 µm, such as from 35 µm to 450 µm, or more preferably from 100 µm to 300 µm.

The term "through-holes for allowing fluid to pass through" refers to openings in a porous structure that enable fluid movement between chambers in the device. These holes may also be designed to restrict the passage of biological cells. In some embodiments, a porous membrane with uniformly distributed micro-scale holes connects two chambers, enabling controlled fluid flow and cell interaction.

The through-holes in the microfluidic device can vary in shape and size. They may be fabricated using methods such as etching, laser drilling, or other suitable techniques. The width of the through-holes typically ranges from 3 µm to 200 µm. In preferred embodiments, the width is between 5 µm and 150 µm. More preferred widths are between 10 µm and 90 µm, with the most preferred range being 20 µm to 90 µm.

Through-holes with widths between 20 µm and 90 µm may facilitate interactions between cells on opposing surfaces. This design can mimic physiological conditions, such as those found in the blood-brain barrier.

The substrate may optionally include integrated conductive lines, which may be embedded within the porous structure to enhance its electrical functionality. Such conductive lines may, for instance, be made of metals like gold, silver, or copper. This enables the structure to function as part of an electronic device or system. In some embodiments, the substrate may also include integrated electrodes, sensors, or actuators positioned on or within the top surface of the porous substrate, the bottom surface, or both. These components may enable functionalities such as electrical stimulation, sensing, or actuation depending on the requirements of the application.

Furthermore, for the porous structure to act as a mask for irradiation or similar processes, it is preferably non-transparent to said light, aside from where the through-holes are situated. This property is advantageous in scenarios where precise patterning of light exposure is necessary, such as in the creation of spatially resolved wettability patterns or other photochemical modifications.

In further embodiments, the substrate may comprise an array of semiconductor islands connected by semiconductor bridges, with the through-holes being the spaces between the islands and bridges. Sensors, actuators, or electrodes may be attached to the top surface or bottom surface of these islands to enhance functionality. The substrate may be a monolithic structure, such as a single silicon wafer. This ensures precise control over the dimensions and arrangement of the through-holes and other features.

As used herein, and unless otherwise specified, the term "light to which the photosensitive inner walls are sensitive" refers to electromagnetic radiation of specific wavelengths that can induce a chemical or physical change in the photosensitive materials forming the inner walls of the microfluidic device. This light prompts a reaction in the photosensitive material, altering surface properties such as wettability. An example includes ultraviolet light in the range of from 100 to 400 nm used to degrade photosensitive silane molecules on the inner walls.

As used herein, and unless otherwise specified, the term "fluidically connected" refers to two or more chambers or components within the microfluidic device that are connected in such a way that fluid can flow between them. Specific embodiments include chambers connected via porous structures with through holes or channels that permit controlled fluid communication.

As used herein, and unless otherwise specified, the term "through holes for allowing fluid to pass through" refers to openings or apertures within the porous structure that enable the movement of fluids between chambers in the microfluidic device. These holes may be sized and arranged to permit fluid flow. In embodiments, they may be sized for restricting the passage of biological cells. An example embodiment includes a porous membrane with uniformly distributed micro-scale holes connecting two chambers.

As used herein, and unless otherwise specified, the term "hydrogel" refers to a three-dimensional network of hydrophilic polymers that can absorb and retain water, forming a gel-like substance. In the context of the microfluidic device, the hydrogel provides a supportive matrix for cell growth and migration. Specific embodiments include hydrogels commonly used in the field, such as those formed from natural or synthetic materials, including but not limited to Collagen-I, fibrin, Matrigel, GeIMA, agarose, acrylamide, or polyethylene glycol diacrylate (PEGDA).

As used herein, and unless otherwise specified, the term "hydrogel precursor solution" refers to a liquid solution containing monomers, oligomers, or pre-polymers that can be cross-linked or polymerized to form a hydrogel. This transformation is typically initiated by a trigger such as light, heat, or a chemical agent. An example includes a solution of acrylamide monomers that polymerize into a polyacrylamide hydrogel upon exposure to ultraviolet light with a photoinitiator.

As used herein, and unless otherwise specified, the expression "initiating polymerization of the hydrogel precursor solution to form the hydrogel" refers to the process of triggering the chemical reaction that converts the liquid precursor solution into a solid or semi-solid hydrogel within the microfluidic device. This can be achieved through various means such as exposure to light of specific wavelengths, increasing temperature, or adding a chemical catalyst. Specific embodiments include initiating polymerization with ultraviolet light in the presence of a photoinitiator, or by adding a cross-linking agent.

As used herein, and unless otherwise specified, the term "second porous structure for retaining hydrogel and ensuring a fluidic connection between the second chamber and the third chamber" refers to a porous barrier that both prevents the hydrogel from moving undesirably between chambers and allows fluid to pass through. This structure balances the need to confine the hydrogel within a specific chamber while maintaining fluid communication. An example embodiment includes a series of (micro-)pillars or a semipermeable membrane that permits fluid flow but restricts the passage of the hydrogel network.

As used herein, and unless otherwise specified, the term "pillars" refers to structural elements within a porous structures of the microfluidic device, typically arranged in arrays or patterns. These pillars create spaces or gaps that allow fluids to pass while providing physical support and limiting the movement of larger entities such as hydrogels or cells. Specific embodiments include microfabricated pillars of defined geometry and spacing, constructed from materials like polydimethylsiloxane (PDMS) or silicon.

As used herein, and unless otherwise specified, the term "hydrophilic" refers to surfaces or materials that have an affinity for water, characterized by low contact angles below 90°. On such surfaces, water tends to spread readily. In the context of the microfluidic device, hydrophilic surfaces promote fluid wetting and can enhance cell adhesion in certain applications.

As used herein, and unless otherwise specified, the term "hydrophobic" refers to surfaces or materials that repel water, characterized by high contact angles above 90°. On such surfaces, water tends to bead up. Hydrophobic surfaces within the microfluidic device can prevent unwanted fluid flow or cell adhesion, influencing fluid dynamics and cell culture conditions.

As used herein, and unless otherwise specified, the term "brain cells" refers to cells derived from neural tissue, including neurons, glial cells, or neural stem cells used in the second chamber for cell culture applications. Specific embodiments may involve culturing primary neurons, astrocytes, or induced pluripotent stem cell-derived neural cells within the microfluidic device.

As used herein, and unless otherwise specified, the term "endothelial cells" refers to cells that line the interior surface of blood vessels and lymphatic vessels, used in embodiments in the first chamber to model vascular structures within the microfluidic device.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

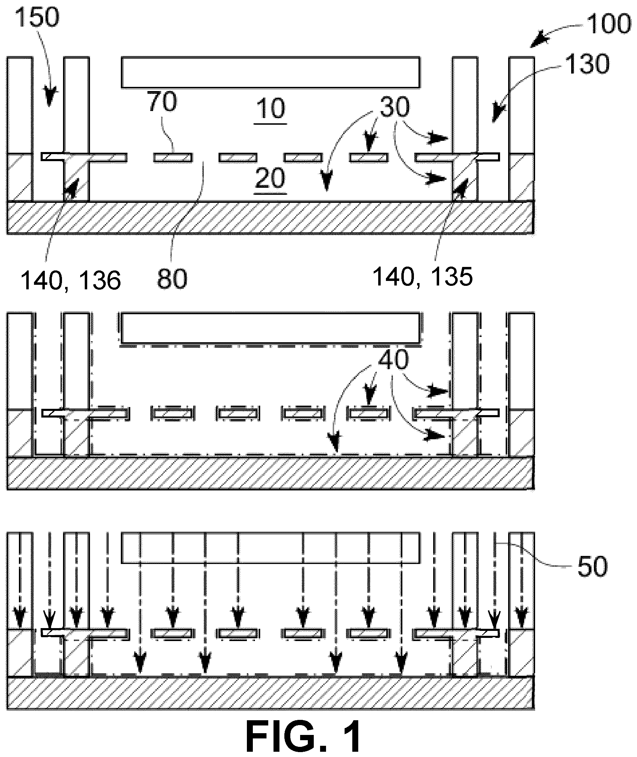

In a first aspect, the present invention relates to a method for forming a microfluidic device (100) for culturing cells. As illustrated Figure 1, second pane, the method comprises:
(a) Providing a microfluidic device (100) comprising:
   - A first chamber (10); and
   - A second chamber (20) fluidically connected to the first chamber (10) by a first porous structure (70) comprising through-holes (80) for allowing fluid to pass through, with the first porous structure (70) physically separating the first chamber (10) and the second chamber (20). Each of these chambers (10, 20) has photosensitive inner walls (40). The photosensitive inner walls (40) are depicted in the second pane of Figure 1 as a dot dash pattern. The transition from the first pane to the second pane of Figure 1 exemplifies how the inner walls (30) can be made photosensitive by the application of a photosensitive coating (40) on inner walls (30).
   As illustrated Figure 1, third pane, the method further comprises:
(b) Irradiating the microfluidic device (100) with light (50) to which the photosensitive inner walls (40) are sensitive, in such a way that the first porous structure (70) acts as a mask during irradiation. This process changes a surface property (e.g., wettability) of the exposed surfaces, imparting a different surface property (e.g., wettability) in the first chamber (10) compared to the second chamber (20).

In embodiments, step (b) may result in exposing a larger proportion of the photosensitive inner walls of the first chamber (10) than those of the second chamber (20) to the light (50). This selective exposure allows for patterning a surface property (e.g., wettability) in different chambers.

Each of the photosensitive inner walls (40) may comprise a photosensitive coating (40), such as one containing photosensitive silane molecules. Using such a coating enables convenient light-based patterning of surface properties. Step (b) may degrade the photosensitive coating, and a greater proportion of the coating may be degraded in the first chamber (10) than in the second chamber (20), resulting, for instance, in a wettability difference between the chambers.

In embodiments, the photosensitive inner walls (40) may be hydrophobic initially, and irradiation may render at least some, preferably all, of the irradiated inner walls hydrophilic after step (b). This hydrophobic-to-hydrophilic transition is useful for controlling fluid behavior and cell interactions. For example, a hydrophobic silane-based coating may be applied to all internal surfaces (30), and selective irradiation may be used to degrade and remove the hydrophobic coating in specific areas. The light (50) used may have a wavelength in the range of from 100 to 400 nm, which is effective for modifying the photosensitive coating.

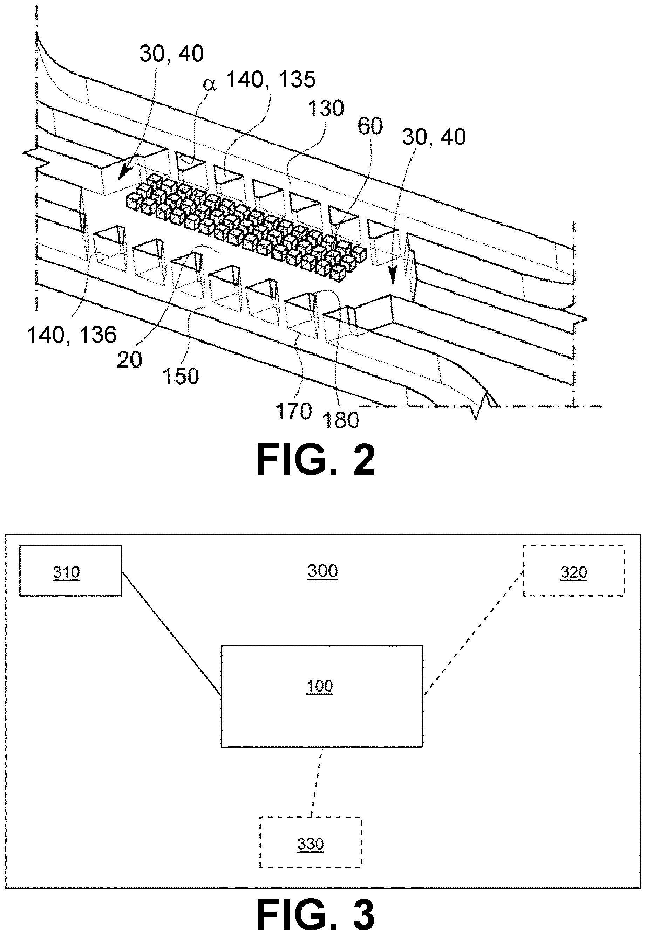

This selective modification of surface property (e.g., wettability) may be used to control fluid behavior within the device, such as guiding hydrogel placement or influencing cell adhesion in specific areas. For instance, as illustrated in Figure 2, when the second chamber (20) is irradiated through the first porous structure (70), the resulting patterned surface properties enable precise spatial control over hydrogel placement and retention. The regions of the second chamber's walls that align with the through-holes (80) of the first porous structure become more hydrophilic upon irradiation, while the masked regions retain their initial hydrophobic character. This patterned wettability creates preferential zones for hydrogel adhesion directly underneath the through-holes, ensuring that the hydrogel (60) remains stably anchored at these locations. Simultaneously, the maintained hydrophobic regions help prevent unwanted hydrogel spreading into the third chamber (130) and fourth chamber (150), which are designed for media flow. This spatial control over hydrogel confinement is particularly advantageous for maintaining distinct functional regions within the device, where the hydrogel-containing zone provides a three-dimensional matrix for cell culture while the adjacent chambers remain open for efficient nutrient delivery and waste removal.

As depicted in Figures 1 and 2, the microfluidic device (100) may further comprise a third chamber (130) separated from the second chamber (20) by a second porous structure (135). This structure is suitable for retaining hydrogel (60) in the second chamber and ensures fluidic connection between the second chamber (20) and the third chamber (130). The first porous structure extends in the third chamber, thereby separating the third chamber into an upper part and a lower part that are fluidically connected. Similarly, a fourth chamber (150) may be separated from the second chamber (20) by a third porous structure (136), allowing for the flow of culture medium and for the diffusion of the culture medium in the second chamber. The first porous structure extends in the fourth chamber, thereby separating the fourth chamber into an upper part and a lower part that are fluidically connected.

The second porous structure (135) and the third porous structure (136) may include a plurality of pillars (140), with spaces between the pillars ensuring fluidic connection while retaining the hydrogel.

The method may further comprise:
(c) Irradiating, after step (b), the third chamber (130) and/or fourth chamber (150) without irradiating the second chamber (20), thereby changing a surface property (e.g., the wettability) of the irradiated surfaces and imparting different surface property (e.g., the wettability) compared to the second chamber (20). For instance, the third and fourth chamber may be hydrophobic before step (c) and become hydrophilic after step (c).

The method may further comprise:
(d) Introducing after step (b) and if performed, after step (c), a hydrogel (60) into the second chamber (20). This hydrogel (60) provides a three-dimensional matrix for culturing cells. Step (d) may involve introducing a hydrogel precursor solution into the second chamber (20) and initiating polymerization to form the hydrogel (60). Polymerization may be triggered by exposing the precursor solution to light, heat, or a chemical agent, offering flexibility in hydrogel formation.

In certain embodiments, the first chamber (10) may contain endothelial cells while the second chamber (20) contains brain cells, which is particularly useful for modeling the blood-brain barrier.

In a second aspect, the invention relates to a microfluidic device (100) for culturing cells, comprising:
- A first chamber (10);
- A second chamber (20) fluidically connected with the first chamber (10) by a first porous structure (70) with through-holes (80), physically separating the chambers;
- Inner walls (30) within each chamber.

A surface property (e.g., the wettability) in the first chamber (10) is different from that in the second chamber (20). This difference may be achieved by providing photosensitive inner walls (40) and irradiating the device (100) with light (50) to which the walls (40) are sensitive, with the first porous structure (70) acting as a mask during irradiation.

As shown in Figures 1 and 2, the device (100) may include a third chamber (130) and a fourth chamber (150), each separated from the second chamber (20) by porous structures (135, 136) designed to retain hydrogel and ensure fluidic connections. The porous structures may comprise pillars (140) to compartmentalize the hydrogel while allowing fluid communication.

The side chambers may be separated from the second chamber (20) by porous structures with spacing between pillars that restrict hydrogel passage, thereby enabling localized hydrogel-based cell culture within the second chamber (20) while maintaining open channels for fluid flow in the side chambers.

In embodiments, the microfluidic device (100) may be constructed from silicon. This helps featuring precisely fabricated porous structures that allow for enhanced control over fluid dynamics and surface properties compared to polymer-based devices. The device may comprise microfabricated channels and chambers with integrated pillars (140) of specific geometries, such as trapezoidal or angular profiles. Such profiles are achievable through silicon fabrication techniques like deep reactive-ion etching (DRIE). The pillars may be designed with dimensions and shapes such as to enable capillary action toward the second chamber (20) and hydrogel retention within the second chamber (20). The silicon material allows for surface functionalization through silanization, providing a range of contact angles and reactivy, and enabling, for instance, fine-tuning of surface wettability for specific experimental requirements. This architecture supports advanced biological modeling, such as recreating physiological barriers with endothelial cells on a silicon mesh (first porous structure (70)) and other cell types within a hydrogel matrix in the second chamber (20).

Different wettability in the third (130) and/or fourth (150) chambers compared to the second chamber (20) enable further customization of the microenvironment. This can be obtained by irradiating these chambers separately after step (b), changing a surface property (e.g., the wettability) of the irradiated surfaces.

The photosensitive inner walls (40) may include a photosensitive coating with silane molecules, allowing for light-based surface property (e.g., the wettability) patterning. Non-irradiated walls may remain hydrophobic, while irradiated walls become hydrophilic, aiding in controlling fluid behavior, gel retention, and/or cell interactions.

In certain configurations, the first chamber (10) may contain endothelial cells, and the second chamber (20) may contain brain cells, effectively mimicking the blood-brain barrier.

In a third aspect, and as illustrated in Figure 3, the invention relates to a system (300) for culturing cells, comprising:
- The microfluidic device (100) as described in the previous embodiments; and
- An injection tool (310) configured to introduce a hydrogel (60) into the second chamber (20).

The injection tool (310) enables precise placement of the hydrogel (60). The system (300) may further include a control tool (320) for managing fluid flow and environmental conditions within the microfluidic device (100), automating and optimizing the cell culture process.

Additionally, the system (300) may comprise a light source (330) configured to irradiate the microfluidic device with light to which the photosensitive inner walls (40) are sensitive, in such a way that porous structure acts as a mask during irradiation. This selective irradiation facilitates surface property (e.g., the wettability) patterning between chambers.

Overall, the invention provides a method and system for creating microfluidic devices with chambers of differing surface property (e.g., the wettability) through selective irradiation of photosensitive inner walls. This approach allows for precise control over cell culture environments, enabling complex biological models such as the blood-brain barrier.

### Example: Selective Hydrogel Loading in a Blood-Brain Barrier Chip

The experiment aimed to develop a method for selectively loading hydrogel into the central channel (second chamber) of a blood-brain barrier (BBB) chip while preventing leakage into the side channels (third and fourth chambers). The BBB chip comprised a silicon-based microfluidic device with a top chamber and a bottom chamber separated by a silicon mesh (first porous structure). The bottom part contained three parallel microfluidic channels, with the central channel (second chamber) designed to hold the hydrogel and brain cells, and the side channels (third and fourth chambers) intended for medium flow to perfuse the hydrogel.

The materials used in the experiment included a silicon-based microfluidic device, silane compounds (tridecafluoro-1,1,2,2-tetrahydrooctyl)trimethoxysilane) for hydrophobic coating, and a UV light source (256 nm wavelength). The experimental setup involved cleaning the silicon device, applying a hydrophobic coating using silane deposition in the vapor phase, and selectively treating specific areas with UV light at 30 mW/cm² for 60 minutes to remove the silane groups and create hydrophilic surfaces. This treatment is performed by irradiating the device from the top, thereby exposing all walls of the first chamber to UV light but irradiating only the parts of the second chamber that are not masked by the silicon mesh.

The experiment is conducted by first filling all channels with the hydrogel precursor. After the selective irradiation of the individual chambers, a hydrogel containing brain cells is injected in liquid form into the central bottom chamber (second chamber). The hydrogel is then polymerized or crosslinked via a pH-, temperature-, or light-induced reaction. Once the hydrogel has polymerized, the lateral channels are utilized to hydrate the hydrogel, supplying nutrients to the embedded cells. Finally, vascular cells are introduced into the upper chamber (first chamber) along with a maintenance medium to support their viability.

The results showed successful selective hydrogel loading in the central channel. The crosslinked hydrogel in the central channel maintained its integrity and showed good retention between the second and third porous structures.

Analysis of the findings indicated that the selective surface modification enabled precise control over hydrogel placement and retention within the microfluidic device.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for forming a microfluidic device (100) for culturing cells, the method comprising:
- (a) providing a microfluidic device (100) comprising:
- a first chamber (10); and
- a second chamber (20) fluidically connected to the first chamber (10) by a first porous structure (70) comprising through holes (80) for allowing fluid to pass through, said first porous structure (70) physically separating the first chamber (10) and the second chamber (20),
- wherein each of said chambers (10, 20) has photosensitive inner walls (40) having a first surface property; and then
- (b) irradiating the microfluidic device (100) with light (50) to which the photosensitive inner walls (40) are sensitive, in such a way that the first porous structure (70) acts as a mask during irradiation, thereby changing the surface property of the exposed surfaces and imparting a different surface property in the first chamber compared to the second chamber.

2. The method according to claim 1, wherein the surface property is the wettability.

3. The method according to claim 1 or claim 2, wherein step (b) results in exposing to said light (50) a larger proportion of the photosensitive inner walls (40) of the first chamber (10) than of the photosensitive inner walls (40) of the second chamber (20).

4. The method according to any one of the preceding claims, wherein each of the photosensitive inner walls (40) comprises a photosensitive coating, such as a photosensitive coating comprising photosensitive silane molecules.

5. The method according to claim 2 or any one of claims 3 to 5 as depending on claim 2, wherein the photosensitive inner walls (40) of a first wettability are hydrophobic and wherein at least some, and preferably all, of the irradiated inner walls become hydrophilic after step (b).

6. The method according to any one of claims 1 to 5, wherein the microfluidic device (100) further comprises a third chamber (130) separated from the second chamber (20) by a second porous structure (135) for retaining hydrogel and ensuring a fluidic connection between the second chamber (20) and the third chamber (130).

7. The method according to claim 6, wherein the microfluidic device (100) further comprises a fourth chamber (150) separated from the second chamber (20) by a third porous structure (136) for retaining hydrogel and ensuring a fluidic connection between the second chamber (20) and the fourth chamber (150).

8. The method according to claim 6 or 7, wherein the second porous structure (135) and/or the third porous structure (136) comprises a plurality of pillars (140), wherein the spaces between the pillars (140) ensure the fluidic connection between the second chamber (20) and the third chamber (130) and/or between the second chamber (20) and the fourth chamber (150).

9. The method according to any one of claims 6 to 8, further comprising:
- (c) irradiating, after step (b), the third and/or fourth chamber (130, 150) without irradiating the second chamber (20), thereby changing a surface property of the irradiated surfaces and imparting a different surface property in the third and/or fourth chamber compared to the second chamber (20).

10. The method according to any one of claims 1 to 9, further comprising:
- (d) introducing, after step (b) and, if performed, after step (c), a hydrogel (60) into the second chamber (20).

11. A microfluidic device (100) for culturing cells, the device comprising:
- a first chamber (10);
- a second chamber (20) fluidically connected with the first chamber (10) by a first porous structure (70) comprising through holes (80) for allowing fluid to pass through, said first porous structure (70) physically separating the first chamber (10) and the second chamber (20);
- each of said chambers (10, 20) having inner walls (30),
- wherein a surface property in the first chamber is different compared to the second chamber.

12. The microfluidic device according to claim 11, further comprising a third chamber (130) separated from the second chamber (20) by a second porous structure (135) for retaining hydrogel and ensuring a fluidic connection between the second chamber (20) and the third chamber (130).

13. The microfluidic device according to claim 12, further comprising a fourth chamber (150) separated from the second chamber (20) by a third porous structure (136) for retaining hydrogel and ensuring a fluidic connection between the second chamber (20) and the fourth chamber (150).

14. The microfluidic device according to claim 12 or 13, wherein the second porous structure (135) and/or the third porous structure (136) comprises a plurality of pillars (140), wherein the spaces between the pillars (140) ensure the fluidic connection between the second chamber (20) and the third chamber (130) and/or between the second chamber (20) and the fourth chamber (150).

15. A system (300) for culturing cells, the system comprising:
- the microfluidic device (100) of any one of claims 11 to 14; and
- an injection tool (310) configured to introduce a hydrogel (60) into the second chamber (20).
